# EUROPÄISCHE PATENTSCHRIFT

(11) **EP 1 959 938 B1**
(45) Veröffentlichungstag und Bekanntmachung des Hinweises auf die Patenterteilung: **27.04.2011**
(21) Anmeldenummer: 06829314.1
(22) Anmeldetag: 05.12.2006
(51) Int. Cl.: A61K 9/70, A61K 31/401, C07K 5/062

(54) **MATRIXKONTROLLIERTES TRANSDERMALES SYSTEM MIT SALZEN DER ACE-HEMMER-DICARBONSÄUREN**
MATRIX-CONTROLLED TRANSDERMAL SYSTEM COMPRISING SALTS OF ACE INHIBITOR DICARBOXYLIC ACIDS
SYSTEME TRANSDERMIQUE MATRICIEL CONTENANT DES SELS DES ACIDES DICARBOXYLIQUES COMME INHIBITEURS DE L'ENZYME DE CONVERSION DE L'ANGIOTENSINE

(30) Priorität: 05.12.2005 DE 102005058166
(43) Veröffentlichungstag der Anmeldung: 27.08.2008
(73) Patentinhaber: Hexal AG, 83607 Holzkirchen (DE)
(72) Erfinder: KLOKKERS, Karin, 83620 Feldkirchen-Westerham (DE); HELFRICH, Michael, 80796 München (DE); NINK, Jörg, 80634 München (DE)
(74) Vertreter: Forstmeyer, Dietmar
(86) Internationale Anmeldenummer: PCT/EP2006/011672
(87) Internationale Veröffentlichungsnummer: WO 2007/065638

(56) Entgegenhaltungen:
- WO-A-02/03970
- WO-A-99/25374
- WO-A-2004/046172
- DE-A1- 19 913 528

## Beschreibung

Die Erfindung betrifft ein stabiles, wirkstoffhaltiges transdermales therapeutisches System zur Anwendung von ACE-Hemmern, deren Metaboliten eine Dicarbonsäure darstellen. Stabile, neutrale Derivate der ACE-Hemmer-Dicarbonsäuren werden durch Salzbildung mit einem organischen Amin und/oder einer Alkaliverbindung erzielt. Bevorzugt werden stabile, neutrale Derivate der ACE-Hemmer-Dicarbonsäuren mit einem organischen Amin, und besonders bevorzugt mit einem Moläquivalent eines organischen Amins erzielt.

Die Langzeittherapie der Hypertonie mit Angiotensin Converting Enzyme-Hemmern (ACE-Hemmer) nimmt einen immer breiteren Raum ein.

ACE-Hemmer sind bei guter Verträglichkeit für ihre zuverlässige Wirksamkeit bekannt. Bisher auf dem Markt erhältlich sind nur Injektibilia oder orale Darreichungsformen der ACE-Hemmer, wie Tabletten oder Kapseln. Nachteilig bei Verwendung der Injektibilia ist die geringe Patienten-Compliance. Bei oralen Darreichungsformen besteht der Nachteil darin, daß der Patient jeden Tag mindestens eine Tablette oder Kapsel schlucken muß und der Blut-Plasmaspiegel immer gewissen Schwankungen unterworfen ist. Ein gleichbleibender Plasmaspiegel ist mit oralen Darreichungsformen kaum zu gewährleisten.

Die transdermale Applikation dagegen bietet für ACE-Hemmer eine Reihe von Vorteilen:
die Haut ist unbegrenzt zugänglich,
es erfolgt kein Milieuwechsel wie bei der peroralen Applikation,
die Handhabung ist einfach und bequem,
es genügt normalerweise eine einmalige Gabe für mindestens 3 Tage statt mehrfacher täglicher Gaben,
die Patienten-Compliance ist wesentlich besser,
es ist eine kontinuierliche Langzeittherapie möglich,
die Freisetzung des Wirkstoffes erfolgt annähernd gemäß einer Kinetik 0-ter Ordnung,
eine Therapie kann schneller unterbrochen werden,
es wird ein konstanter Plasmaspiegel über längere Zeit sichergestellt,
ein anfangs zu hoher Plasmaspiegel, wie bei intravenöser Applikation wird vermieden und
aufgrund der Umgehung der 1. Passage bedarf es teilweise einer niedrigeren Dosierung als bei der oralen Gabe, wodurch eine geringere Nebenwirkungsrate auftritt und
die Gefahr von Über- oder Unterdosierung geringer ist.

In EP 0 439 430 wird ein transdermales Reservoirsystem mit einem Gehalt an einem ACE-Hemmer beschrieben, welches a) eine wirkstoffundurchlässige Deckschicht (closed outer layer), b) ein wirkstoffhaltiges Reservoir mit einem Vehikel bzw. Lösungsmittel sowie ggf. einer Membran, c) eine Kleberschicht und d) eine abziehbare Schutzschicht (peel off protective layer) enthält. Als Wirkstoff dient ein Salz eines Zwitterions, wobei als Zwitterion beispielsweise Benazeprilat und als salzbildende Komponente beispielsweise Chlorprocain, Cholin, Diethanolamin, Ethylendiamin, Methylglucamin, Procain oder Alkalimetallhydroxide, -carbonate oder -hydrogencarbonate beispielsweise von Lithium oder Kalium vorgesehen sein können und als Beispiele für Disalze Dilithium- und Dikaliumbenazeprilat genannt werden (Tabelle).

EP 0 452 837 beschreibt eine Matrix für Pflaster, die u.a. ACE-Hemmer als Wirkstoffe enthält. Als mögliche ACE-Hemmer werden Delaprilhydrochlorid, Enalaprilmaleat, Captopril, Alacepril und (R )-3-[(S)-1-Carboxy-5-(4-piperidyl)-pentyl]-amino-4-oxo-2,3,4,5-tetrahydro-1,5-benzothiazepin-5-Essigsäure (=Dicarbonsäure) genannt. Basische Substanzen werden hier nur in sehr geringer Menge als Solubilisatoren eingesetzt.

WO 96/29 999 offenbart ein TTS mit einer Matrix auf Basis von Polyisobutylen oder Butylkautschuk mit einem Gehalt an Trandolapril und/ oder Ramipril.

In WO 02/03 970 wird ein Matrix-TTS beschrieben, dessen Matrixschicht einen ACE-Hemmer in Form einer Dicarbonsäure enthält, die zu einem Derivat aus folgender Gruppe derivatisiert ist: Diester, mit Basen erhältliches Di-salz und mit Säuren erhältliches Mono-salz. Es hat sich jedoch gezeigt, daß ein TTS mit einem Di-salz einer Base eine hohe Hautirritation zeigt.

ACE-Hemmer zeigen häufig eine geringe Stabilität und können verschiedene Abbaureaktionen eingehen. ACE-Hemmer-Dicarbonsäuren mit dem folgenden Strukturelement können sich beispielsweise zu substituierten Diketopiperazinen umlagern. Über einen nukleophilen Angriff des Stickstoffatoms kommt es hierbei zu einer intramolekularen Lactambildung wie folgende Gleichung veranschaulicht:

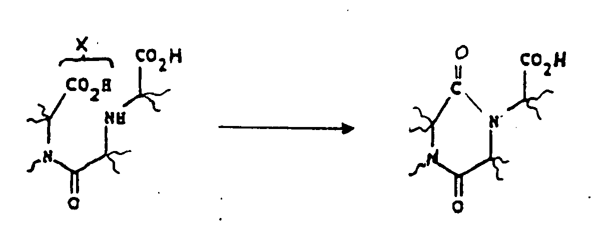

Die intramolekulare Lactambildung kann durch Zusatz von Säuren, welche die sekundäre Amingruppe blockieren können, verhindert werden. Die so mit Säuren gebildeten Salze der ACE-Hemmer-Dicarbonsäuren (s. WO 02/03970) haben den Nachteil, daß sich durch den niedrigen pH-Wert ein erhöhtes Hautirriationspotential ergibt.

Verwendet man Di-salze der ACE-Hemmer-Dicarbonsäuren mit Basen (s. WO 02/03970), so wird ebenfalls eine intramolekulare Lactambildung verhindert, jedoch zeigt die gesamte Formulierung einen basischen pH-Wert, der ebenfalls zu Hautirritationen führen kann.

Die Aufgabe der vorliegenden Erfindung ist die Bereitstellung eines Matrix-TTS mit einem Gehalt an Salzen von ACE-Hemmer-Dicarbonsäuren, mit hoher Stabiltät und geringem Hautirritationspotential. Der Wirkstoffgehalt soll über einen längeren Zeitraum stabil sein und möglichst geringen Zersetzungsvorgängen unterworfen sein. Das transdermale System soll einen hohen Flux zeigen, d.h. der Wirkstoff soll mit einer hohen Hautpermeationsrate abgegeben werden.

Die der Erfindung zugrundeliegende Aufgabe wird dadurch gelöst, dass ein Salz, bevorzugt ein Monosalz, einer ACE-Hemmer-Dicarbonsäure mit einem organischen Amin und/oder einer Alkaliverbindung vorgesehen wird.

Das erfindungsgemäße Salz kann mit einem Monoamin als organischem Amin vorgesehen sein.

So kann das erfindungsgemäße Salz mit einem primären Amin, einem sekundären oder einem tertiären Amin als organischem Amin vorgesehen sein.

So kann das erfindungsgemäße Salz mit einem aliphatischen primären C₄₋₁₂-Amin vorgesehen sein.

So kann das erfindungsgemäße Salz mit Butylamin, Pentylamin, Hexylamin, Heptylamin, Octylamin, Nonylamin, Decylamin, Undecylamin, Dodecylamin oder Trometamol (= 2-Amino-2-hydroxymethyl-1,3-propandiol) als aliphatischem primären C₄₋₁₂-Amin vorgesehen sein.

Auch kann das erfindungsgemäße Salz mit Pyrrolidon oder einem seiner Derivate als sekundärem Amin vorgesehen sein.

Auch kann das erfindungsgemäße Salz mit Triethanolamin als tertiärem Amin vorgesehen sein.

Das erfindungsgemäße Salz kann mit einer Alkaliverbindung vorgesehen sein, wobei eine Alkaliverbindung im Sinne dieser Verbindung jede Verbindung ist, die ein Alkalimetallkation umfasst. Bevorzugt werden so Salze der ACE-Hemmer-Dicarbonsäure mit einem Alkalimetallkation gebildet, die auch Alkalisalze der ACE-Hemmer-Dicarbonsäure genannt werden. Beispiele für geeignete Alkalimetallkationen sind Lithium-, Natrium-, Kalium-, Caesium- oder Rubidiumkationen, von denen Lithium-, Natrium- und Kaliumkationen besonders bevorzugt sind. Auch Ammonium (NH₄⁺) ist als ein Kation bevorzugt (Pseudo-Alkalimetallkation). Bevorzugt hat das Gegenanion der Alkaliverbindung, insbesondere des Alkalimetallkations, Protonenakzeptoreigenschaften, und die Alkaliverbindung ist besonders bevorzugt ein Alkalimetallkationen umfassendes Salz einer starken oder schwachen, anorganischen oder organischen Säure. Bevorzugte Beispiele sind Alkaliverbindungen wie Alkalimetallhydroxide, wie zum Beispiel Lithiumhydroxid, Natriumhydroxid oder Kaliumhydroxid, Alkalimetallalkoholate, wie zum Beispiel Lithiummethanolat, Lithiumethanolat, Natriummethanolat, Natriumethanolat, Kaliummethanolat oder Kaliumethanolat, oder Alkalimetallcarbonate, wie zum Beispiel Natriumcarbonat oder Kaliumcarbonat, oder Alkalimetallhydrogencarbonate, wie zum Beispiel Natriumhydrogencarbonat oder Kaliumhydrogencarbonat, oder Alkalimetalltartrate, wie zum Beispiel Natriumtartrat oder Kaliumtartrat, oder Alkalimetallmaleate, wie zum Beispiel Natriummaleat oder Kaliummaleat. Als Ammonium-Verbindung bevorzugt ist Ammoniumhydroxid.

Das erfindungsgemäße Salz kann mit einer Erdalkaliverbindung vorgesehen sein, wobei eine Erdalkaliverbindung im Sinne dieser Verbindung jede Verbindung ist, die ein Erdalkalimetallkation umfasst. Bevorzugt werden so Salze der ACE-Hemmer-Dicarbonsäure mit einem Erdalkalimetallkation gebildet, die auch Erdalkalisalze der ACE-Hemmer-Dicarbonsäure genannt werden. Beispiele für geeignete Erdalkalimetallkationen sind Magnesium-, oder Calciumkationen. Bevorzugt hat das Gegenanion der Erdalkaliverbindung, insbesondere des

Erdalkalimetallkations, Protonenakzeptoreigenschaften, und die Erdalkaliverbindung ist besonders bevorzugt ein Erdalkalimetallkationen umfassendes Salz einer starken oder schwachen, anorganischen oder organischen Säure. Bevorzugte Beispiele sind Erdalkaliverbindungen wie Erdalkalimetallhydroxide, wie zum Beispiel Magnesiumhydroxid oder Calciumhydroxid, oder Erdalkalimetallcarbonate, wie zum Beispiel Magnesiumcarbonat oder Calciumcarbonat. Im Fall eines erfindungsgemäßen Salzes mit einer Erdalkaliverbindung beträgt das Molverhältnis ACE-Hemmer-Dicarbonsäure : Erdalkaliverbindung bevorzugt 1 : 0,5 bis 1 : kleiner 1, weiter bevorzugt 1 : 0,5 bis 1 : 0,9, insbesondere 1 : 0,5 bis 1 : 0,55.

Das erfindungsgemäße Salz umfasst ein Salz mit einem oder mehreren organischen Amin(en), bevorzugt einem, zwei oder drei organischen Amin(en), oder einer oder mehreren Alkaliverbindung(en), bevorzugt einer, zwei oder drei Alkaliverbindung(en), oder einer oder mehreren Erdalkaliverbindung(en), bevorzugt einer, zwei oder drei Erdalkaliverbindung(en), sowie beliebige Mischungen davon. Bevorzugt umfasst das erfindungsgemäße Salz ein Salz mit zwei voneinander unterschiedlichen organischen Aminen, oder zwei voneinander unterschiedlichen Alkaliverbindungen, oder zwei voneinander unterschiedlichen Erdalkaliverbindungen, oder einem organischen Amin und einer Alkaliverbindung, und dergleichen.

Bei dem erfindungsgemäßen Salz kann es sich um ein Salz einer ACE-Hemmer-Dicarbonsäure aus der Gruppe der Dicarbonsäuren von Imidapril, Fosinopril, Moexipril, Perindopril, Spirapril, Benazepril, Cilazapril, Lisinopril, Quinapril, Enalapril, Delapril, Ramipril und Trandolapril handeln.

So kann ein erfindungsgemäßes Salz einer ACE-Hemmer-Dicarbonsäure von Trandolapril oder Ramipril vorgesehen sein.

Für das erfindungsgemäße Salz kann das Molverhältnis von ACE-Hemmer-Dicarbonsäure : Amin oder ACE-Hemmer-Dicarbonsäure: Alkaliverbindung von 1: kleiner 2 betragen.

So kann für das erfindungsgemäße Salz das Molverhältnis von ACE-Hemmer-Dicarbonsäure : Amin oder ACE-Hemmer-Dicarbonsäure : Alkaliverbindung von 1 : 0,5 bis 1 : kleiner 2, bevorzugt 1 : 0,5 bis 1 : 1,9, weiter bevorzugt 1: 0,9 bis 1 : 1,5, weiter bevorzugt 1 : 1 bis 1,5, besonders bevorzugt 1 : 1,1 und insbesondere von etwa 1 : 1 betragen.

Die der Erfindung zugrundeliegenden Aufgabe wird ferner gelöst durch ein transdermales therapeutisches System mit mindestens einem Salz einer ACE-Hemmer-Dicarbonsäure mit mindestens einem organischen Amin und/oder mindestens einer Alkaliverbindung als Wirkstoff. Besonders bevorzugt wird die der Erfindung zugrundeliegenden Aufgabe durch ein transdermales therapeutisches System mit mindestens einem Monosalz einer ACE-Hemmer-Dicarbonsäure mit mindestens einem organischen Amin und/oder mindestens einer Alkaliverbindung als Wirkstoff gelöst, insbesondere mit einem Monosalz einer ACE-Hemmer-Dicarbonsäure mit einem organischen Amin als Wirkstoff.

Dabei kann für das erfindungsgemäße transdermale therapeutische System ein Monoamin als organisches Amin vorgesehen sein.

So kann für das erfindungsgemäße transdermale therapeutische System ein primäres Amin, ein sekundäres Amin oder tertiäres Amin als organisches Amin vorgesehen sein.

So kann für das erfindungsgemäße transdermale therapeutische System ein aliphatisches primäres C₄₋₁₂-Amin vorgesehen sein.

So kann für das erfindungsgemäße transdermale therapeutische System Butylamin, Pentylamin, Hexylamin, Heptylamin, Octylamin, Nonylamin, Decylamin, Undecylamin, Dodecylamin, oder Trometamol (= 2-Amino-2-hydroxymethyl-1,3-propandiol) als aliphatisches primäres C₄₋₁₂-Amin vorgesehen sein.

Ferner kann das erfindungsgemäße transdermale therapeutische System mit Pyrrolidon oder einem seiner Derivate als sekundärem Amin vorgesehen sein.

Ferner kann das erfindungsgemäße transdermale therapeutische System mit Triethanolamin als tertiärem Amin vorgesehen sein.

Ferner kann das erfindungsgemäße transdermale therapeutische System mit einer Alkaliverbindung vorgesehen sein, wobei eine Alkaliverbindung im Sinne dieser Verbindung jede Verbindung ist, die ein Alkalimetallkation umfasst. Beispiele für geeignete Alkalimetallkationen sind Lithium-, Natrium-, Kalium-, Caesium- oder Rubidiumkationen, von denen Lithium-, Natrium- und Kaliumkationen besonders bevorzugt sind. Auch Ammonium (NH₄⁺) ist als ein Kation bevorzugt (Pseudo-Alkalimetallkation). Bevorzugt hat das Gegenanion der Alkaliverbindung, insbesondere des Alkalimetallkations, Protonenakzeptoreigenschaften, und die Alkaliverbindung ist besonders bevorzugt ein Alkalimetallkationen umfassendes Salz einer starken oder schwachen, anorganischen oder organischen Säure. Bevorzugte Beispiele sind Alkaliverbindungen wie Alkalimetallhydroxide, wie zum Beispiel Lithiumhydroxid, Natriumhydroxid oder Kaliumhydroxid, Alkalimetallalkoholate, wie zum Beispiel Lithiummethanolat, Lithiumethanolat, Natriummethanolat, Natriumethanolat, Kaliummethanolat oder Kaliumethanolat, oder Alkalimetallcarbonate, wie zum Beispiel Natriumcarbonat oder Kaliumcarbonat, oder Alkalimetallhydrogencarbonate, wie zum Beispiel Natriumhydrogencarbonat oder Kaliumhydrogencarbonat, oder Alkalimetalltartrate, wie zum Beispiel Natriumtartrat oder Kaliumtartrat, oder Alkalimetallmaleate, wie zum Beispiel Natriummaleat oder Kaliummaleat. Als Ammonium-Verbindung bevorzugt ist Ammoniumhydroxid.

Ferner kann das erfindungsgemäße transdermale therapeutische System mit einer Erdalkaliverbindung vorgesehen sein, wobei eine Erdalkaliverbindung im Sinne dieser Verbindung jede Verbindung ist, die ein Erdalkalimetallkation umfasst. Beispiele für geeignete Erdalkalimetallkationen sind Magnesium-, oder Calciumkationen. Bevorzugt hat das Gegenanion der Erdalkaliverbindung, insbesondere des Erdalkalimetallkations, Protonenakzeptoreigenschaften, und die Erdalkaliverbindung ist besonders bevorzugt ein Erdalkalimetallkationen umfassendes Salz einer starken oder schwachen, anorganischen oder organischen Säure. Bevorzugte Beispiele sind Erdalkaliverbindungen wie Erdalkalimetallhydroxide, wie zum Beispiel Magnesiumhydroxid oder Calciumhydroxid, oder Erdalkalimetallcarbonate, wie zum Beispiel Magnesiumcarbonat oder Calciumcarbonat. Im Fall eines erfindungsgemäßen Salzes mit einer Erdalkaliverbindung beträgt das Molverhältnis ACE-Hemmer-Dicarbonsäure : Erdalkaliverbindung bevorzugt 1 : 0,5 bis 1 : kleiner 1, weiter bevorzugt 1 : 0,5 bis 1 : 0,9, insbesondere 1 : 0,5 bis 1 : 0,55.

Ferner kann das erfindungsgemäße transdermale therapeutische System mit einem oder mehreren organischen Amin(en), bevorzugt einem, zwei oder drei organischen Amin(en), oder einer oder mehreren Alkaliverbindung(en), bevorzugt einer, zwei oder drei Alkaliverbindung(en), oder einer oder mehreren Erdalkaliverbindung(en), bevorzugt einer, zwei oder drei Erdalkaliverbindung(en), sowie beliebige Mischungen davon, vorgesehen sein. Bevorzugt umfasst das erfindungsgemäße Salz ein Salz mit zwei voneinander unterschiedlichen organischen Aminen, zwei voneinander unterschiedlichen Alkaliverbindungen, oder zwei voneinander unterschiedlichen Erdalkaliverbindungen, oder einem organischen Amin und einer Alkaliverbindung, und dergleichen.

Ferner kann das erfindungsgemäße transdermale therapeutische System mit mindestens einem Salz einer ACE-Hemmer-Dicarbonsäure aus der Gruppe der Dicarbonsäuren von Imidapril, Fosinopril, Moexipril, Perindopril, Spirapril, Benazepril, Cilazapril, Lisinopril, Quinapril, Enalapril, Delapril, Ramipril und Trandolapril vorgesehen sein.

Bevorzugt kann das erfindungsgemäße transdermale therapeutische System mit mindestens einem Monosalz einer ACE-Hemmer-Dicarbonsäure aus der Gruppe der Dicarbonsäuren von Imidapril, Fosinopril, Moexipril, Perindopril, Spirapril, Benazepril, Cilazapril, Lisinopril, Quinapril, Enalapril, Delapril, Ramipril und Trandolapril, oder Mischungen von zwei, drei oder mehreren davon, vorgesehen sein.

So kann das erfindungsgemäße transdermale therapeutische System mit einem Salz einer ACE-Hemmer-Dicarbonsäure von Trandolapril und/oder Ramipril vorgesehen sein.

Bevorzugt kann das erfindungsgemäße transdermale therapeutische System mit einem Monosalz einer ACE-Hemmer-Dicarbonsäure von Trandolapril und/oder Ramipril vorgesehen sein.

Für das erfindungsgemäße transdermale therapeutische System kann das Molverhältnis von ACE-Hemmer-Dicarbonsäure : Amin oder ACE-Hemmer-Dicarbonsäure : Alkaliverbindung von 1 : kleiner 2 betragen.

So kann für das erfindungsgemäße transdermale therapeutische System das Molverhältnis von ACE-Hemmer-Dicarbonsäure : Amin oder ACE-Hemmer-Dicarbonsäure: Alkaliverbindung von 1 : 0,5 bis 1 : kleiner 2, bevorzugt 1 : 0,5 bis 1 : 1,9, weiter bevorzugt 1 : 0,9 bis 1 : 1,5, weiter bevorzugt 1 : 1 bis 1,5, besonders bevorzugt 1 : 1,1 und insbesondere von etwa 1 : 1 betragen.

Das erfindungsgemäße transdermale therapeutische System kann mit
- einer wirkstoffundurchlässigen Deckschicht,
- einer oder mehreren wirkstoffhaltigen selbstklebenden Matrixschichten und
- einer abziehbaren Schutzschicht
vorgesehen sein.

Ferner kann das erfindungsgemäße transdermale therapeutische System mit
- einer wirkstoffundurchlässigen Deckschicht,
- einer oder mehreren wirkstoffhaltigen Matrixschichten,
- die applikationsseitig mit einer Haftkleberschicht versehen ist (sind), und
- einer abziehbaren Schutzschicht
vorgesehen sein.

So kann das erfindungsgemäße transdermale therapeutische System mit einer nicht-selbstklebenden Matrixschicht und einer separaten Schicht eines Haftklebers vorgesehen sein.

Für das erfindungsgemäße transdermale therapeutische System können der oder die Wirkstoffe, d.h. ein oder mehrere Salze, insbesondere ein oder mehrere Monosalze, einer ACE-Hemmer-Dicarbonsäure mit mindestens einem organischen Amin und/oder mindestens einer Alkaliverbindung, in der Matrix gelöst und/oder in Form von Emulsionströpfchen vorliegen.

Für das erfindungsgemäße transdermale therapeutische System kann der Gehalt an ACE-Hemmer-Dicarbonsäure 2 bis 35 Gew.-% betragen, bezogen auf das Matrixgewicht.

So kann für das erfindungsgemäße transdermale therapeutische System der Gehalt an ACE-Hemmer-Dicarbonsäure 10 bis 25 Gew.-% betragen, bezogen auf das Matrixgewicht.

Das erfindungsgemäße transdermale therapeutische System kann mit einem druckempfindlichen Klebemittel auf Basis Polyurethan, Polyisobutylen, Polyvinylether, Polyacrylat, Silikon, Styrol-Block-Copolymer- oder- eines Gemischs derselben vorgesehen sein.

So kann das erfindungsgemäße transdermale therapeutische System mit einem druckempfindlichen Klebemittel auf Basis Styrol-Isopren-Styrol-Block-Copolymer (SIS) oder Styrol-Butadien-Styrol-Block-Copolymer vorgesehen sein.

Auch kann das erfindungsgemäße transdermale therapeutische System mit einem druckempfindlichen Klebemittel auf Basis von Polyacrylat oder Polyisobutylen vorgesehen sein.

Ferner kann das erfindungsgemäße transdermale therapeutische System mit einem Matrixbildner aus der Gruppe von Polyacrylat, Polyisobutylen, Silicon, Styrol-Block-Copolymer oder einem ihrer Gemische vorgesehen sein.

So kann das erfindungsgemäße transdermale therapeutische System mit einem Styrol-Isopren-Styrol-Block-Coploymer (SIS) als Matrixbildner vorgesehen sein. '

Auch kann das erfindungsgemäße transdermale therapeutische System mit einer selbstklebenden Matrix auf Basis von Polyacrylat vorgesehen sein.

Ferner kann das erfindungsgemäße transdermale therapeutische System mit einem Haftkleber und/oder einer Matrix auf Polyacrylatbasis vorgesehen sein, bei der es sich um ein Homopolymer, Copolymer oder Terpolymer handelt.

So kann das erfindungsgemäße transdermale therapeutische System mit einem Haftkleber und/oder einer Matrix auf Polyacrylatbasis vorgesehen sein, enthaltend oder bestehend aus verschiedenen Acrylsäurederivaten.

So kann das erfindungsgemäße transdermale therapeutische System mit einem Haftkleber und/oder einer Matrix auf Polyacrylatbasis, bestehend aus Acrylatpolymer aus
- mindestens 50 Gew.-% eines Acrylat-, Methacrylat-, Alkylacrylat-, Alkylmethacrylat- oder Acrylamid-Monomers,
- 0 bis 20 Gew.-% eines funktionellen Monomers, copolymerisierbar mit Acrylat, und
- 0 bis 50 Gew.-% eines anderen Monomeren
vorgesehen sein.

Ferner kann das erfindungsgemäße transdermale therapeutische System mit einem Permeationsförderer aus der durch
gesättigte und/oder ungesättigte Fettalkohole mit jeweils 8 bis 18 C-Atomen;
Teebaumöl;
gesättigte und/oder ungesättigte cyclische Ketone; Alkyl-Methylsulfoxide;
gesättigte und/oder ungesättigte Fettsäuren mit jeweils 8 bis 18 C-Atomen;
Ester gesättigter und/oder ungesättigter Fettsäuren mit jeweils 8 bis 18 C-Atomen;
Salze gesättigter und/oder ungesättigter Fettsäuren mit jeweils 8 bis 18 C-Atomen;
natürliches Vitamin E;
synthetisches Vitamin E und/oder Vitamin E- Derivate;
Sorbitanfettsäureester;
ethoxylierte Sorbitanfettsäureester;
Azone, insbesondere Laurocapram;
1-Alkylpyrrolidon;
Blockcopolymere von Polyethylenglykol und Dimethylsiloxan mit kationischer Gruppe an einem Ende;
Polyoxyethylen-10-stearylether;
Gemisch aus Polyoxyethylen-10-stearylether und
Glyceryldilaurat;
Dodecyl-2-(N,N-dimethylamino)-propanoltetradecanoat und/oder Dodecyl-2-(N,N-dimethylamino)-propionat;
N-Acetylprolinatester (N-Acetyl-pyrrolidon-2-carbonsäure-ester) mit > 8 C-Atomen;
nichtionische Tenside, insbesondere Laurylether;
Ester von Polyoxyethylen;
Dimethyl(arylimino)sulfuran;
Gemisch aus Ölsäureanalogon(a) und Propylenglykol;
Gemisch aus Padimat O, Octylsalicylat, Isopropylmyristat, Isopropylpalmitat, Octylmethoxycinnamat, Laurocapram;
hochdisperses Siliziumdioxid (Aerosil^{®});
Polyoxyethylen-7-glycerol-monococoat (Cetiol^{®} HE);
2-Octyldodecanol (Eutanol^{®} G);
und deren Gemischen
gebildeten Gruppe vorgesehen sein.

So kann das erfindungsgemäße transdermale therapeutische System mit Polyoxyethylen-7-glycerol-monococoat (Cetiol^{®} HE) oder 2-Octyldodecanol (Eutanol^{®} G) als Permeationsförderer vorgesehen sein.

Für das erfindungsgemäße transdermale therapeutische System kann der Gehalt an Klebemittel der selbstklebenden Matrix 20 bis 90 Gew.-%, bevorzugt 30 bis 80 Gew.-% und insbesondere 40 bis 60 Gew.-% betragen, Rest Wirkstoff(e), fakultativer Permeationsförderer und fakultativer Füllstoff, jeweils bezogen auf das Matrixgewicht.

Schließlich wird die der Erfindung zugrundeliegende Aufgabe durch ein Verfahren zur Herstellung eines erfindungsgemäßen transdermalen therapeutischen Systems gelöst, bei dem das (die) organische(n) Amin(e) und die ACE-Hemmer-Dicarbonsäure(n) zusammen in die Matrix-Lösung bzw. Suspension eingearbeitet werden und das (die) Amin-Salz(e) in der Matrix-Lösung bzw. Suspension in-situ gebildet wird (werden).

Bei dem erfindungsgemäßen Verfahren zur Herstellung eines erfindungsgemäßen transdermalen therapeutischen Systems kann (können) das (die) Amin-Salz(e) der ACE-Hemmer-Dicarbonsäure(n) direkt in die Matrix eingebracht werden.

Ebenso stellt die Erfindung ein Verfahren zur Herstellung eines erfindungsgemäßen transdermalen therapeutischen Systems bereit, bei dem die Alkaliverbindung(en) und die ACE-Hemmer-Dicarbonsäure(n) zusammen in die Matrix-Lösung bzw. Suspension eingearbeitet werden und das (die) Alkaliverbindungs-Salz(e) in der Matrix-Lösung bzw. Suspension in-situ gebildet wird (werden).

Bei dem erfindungsgemäßen Verfahren zur Herstellung eines erfindungsgemäßen transdermalen therapeutischen Systems kann (können) das (die) Alkaliverbindungs-Salz(e) der ACE-Hemmer-Dicarbonsäure(n) direkt in die Matrix eingebracht werden.

Die Matrix kann im Verlauf eines jeden erfindungsgemäßen Herstellungsverfahrens des erfindungsgemäßen transdermalen therapeutischen Systems auch als Lösung oder Suspension vorliegen; das bzw. die Lösungsmittel bzw. Suspensionsmittel können in einem späteren Verfahrensschritt gegebenenfalls abgedampft werden.

Überraschenderweise wurde also nun gefunden, daß die Salze von ACE-Hemmer-Dicarbonsäuren, die durch Umsetzung mit einem organischen Amin und/oder einer Alkaliverbindung gebildet werden, weitgehend stabil gegen Zersetzung sind, insbesondere gegenüber intramolekularer Lactambildung. Überraschenderweise wurde auch gefunden, daß die Salze von ACE-Hemmer-Dicarbonsäuren, die durch Umsetzung mit einem Moläquivalent eines organischen Amins und/oder einer Alkaliverbindung gebildet werden, weitgehend besonders stabil gegen Zersetzung sind, insbesondere gegenüber intramolekularer Lactambildung. Die intramolekulare Lactambildung wird durch eine Blockierung der Carboxylgruppe X (s. Gleichung) durch das Amin bzw. die Alkaliverbindung verhindert. Da die Carboxylatgruppe X die acidere Carboxylatgruppe in der ACE-Hemmer-Dicarbonsäure ist, kann die intramolekulare Lactambildung bereits durch ein Moläquivalent Amin oder Alkaliverbindung verhindert werden. Die Salze, insbesondere die Mono-salze, der ACE-Hemmer-Dicarbonsäuren liegen in einem für die Haut neutralen Bereich vor und besitzen damit verbunden ein minimales Hautirritationspotential. Aus einem Matrixbildner werden die Amin-Salze oder Alkaliverbindungs-Salze der ACE-Hemmer-Dicarbonsäuren mit einem hohen Flux abgegeben.

Bevorzugt liegt der pH-Wert, der sich bei Applikation des transdermalen therapeutischen Systems mit einem erfindungsgemäßen Salz, insbesondere einem Monosalz, der ACE-Hemmer-Dicarbonsäure mit einem organischen Amin und/oder einer Alkaliverbindung einstellt, zwischen 5,0 und 7,5, bevorzugt von 5,0 bis 7,0, und besonders bevorzugt von 5,0 bis 6,8. Dieser pH-Wert kann näherungsweise bestimmt werden, indem man das transdermale therapeutische System in einer geeigneten Menge Wasser, beispielsweise 25 ml, hinreichend lange schüttelt und, zum Beispiel nach Ablauf von 2 Stunden, den pH-Wert mit einem üblichen Messverfahren misst. Ein derartiger pH-Wert-Bereich ist besonders vorteilhaft, da der pH-Wert der Haut bei etwa 4,9 - 5,5 liegt.

Überraschend wurde auch gefunden, dass ein erfindungsgemäßes transdermales therapeutisches System mit einem erfindungsgemäßen Salz einer ACE-Hemmer-Dicarbonsäure mit einem organischen Amin und/oder einer Alkaliverbindung besonders stabil, beispielsweise bei verlängerter Lagerung, ist. Darüber hinaus zeichnet sich ein erfindungsgemäßes transdermales therapeutisches System mit einem erfindungsgemäßen Salz einer ACE-Hemmer-Dicarbonsäure mit einem organischen Amin und/oder einer Alkaliverbindung durch einen überraschend hohen Flux (Permeation) des Wirkstoffes bei der Applikation aus, der beispielsweise höher ist, als der der freien ACE-Hemmer-Dicarbonsäure. Diese Effekte sind besonders ausgeprägt bei dem erfindungsgemäßen transdermalen therapeutischen System mit dem erfindungsgemäßen Salz einer ACE-Hemmer-Dicarbonsäure mit einem organischen Amin, insbesondere dem erfindungsgemäßen Monosalz einer ACE-Hemmer-Dicarbonsäure mit einem organischen Amin.

Das erfindungsgemäße transdermale therapeutische System besteht aus einer für den Wirkstoff undurchlässigen Deckschicht (1), aus einer oder mehreren den Wirkstoff und/ oder fakultative Permeationsförderer enthaltenden, selbstklebenden Matrixschicht(en) (2) oder einer oder mehreren Matrixschicht(en) (4), die mit einem Haftkleber (5) beschichtet sind und einer abziehbaren Schutzschicht (3).

In dem erfindungsgemäßen transdermalen therapeutischen System kann mindestens ein stabiles Amin-Salz einer ACE-Hemmer-Dicarbonsäure, das auf einer Reaktion eines organischen Amins mit einer ACE-Hemmer-Dicarbonsäure basiert, verwendet werden. Als organisches Amin werden bevorzugt primäre, aliphatische Amine mit 4 bis 12 C-Atomen eingesetzt. Besonders bevorzugt werden Amin mit einer Aminogruppe (= Monoamin) verwendet. Beispielsweise können Butylamin, Pentylamin, Hexylamin, Heptylamin, Octylamin, Nonylamin, Decylamin, Undecylamin, Dodecylamin oder Trometamol (=2-Amino-2-hydroxymethyl-1,3-propandiol) eingesetzt werden. Insbesondere wird als primäres, aliphatisches Amin Trometamol verwendet. Es können auch sekundäre Amine wie Pyrrolidon oder dessen Derivate eingesetzt werden. Ebenso können tertiäre Amine wie Triethanolamin verwendet werden.

Das erfindungsgemäße transdermale therapeutische System kann als Wirkstoff die ACE-Hemmer enthalten, deren aktive Metaboliten eine Dicarbonsäure darstellen, wie z.B. Imidapril, Fosinopril, Moexipril, Perindopril, Spirapril, Benazepril, Cilazapril, Lisinopril, Quinapril, Enalapril, Delapril, Ramipril und/ oder Trandolapril. Unter einer Dicarbonsäure eines ACE-Hemmers versteht man den aktiven Metaboliten des ACE-Hemmers, bei welchem die im Molekül vorhandene Carbonsäureester-Gruppierung verseift wurde. Demnach zählen zu den ACE-Hemmer-Dicarbonsäure z.B. Imidaprilat, Fosinoprilat, Moexiprilat, Perindoprilat, Spiraprilat, Benazeprilat, Cilazaprilat, Lisinoprilat, Quinaprilat, Enalaprilat, Delaprilat, Ramiprilat und/ oder Trandolaprilat. Bevorzugt werden die Alkaliverbindungs- oder Amin-Salze von Trandolaprilat und/ oder Ramiprilat als Wirkstoffkomponenten verwendet, wobei die Aminsalze bevorzugt werden.

Bei dem erfindungsgemäßen Amin-Salz handelt es sich um ein Salz, bevorzugt ein Mono-Salz, in welchem das Mol-Verhältnis von ACE-Hemmer-Dicarbonsäure zu Amin kleiner als 1:2, insbesondere 1:0,5 bis 1:<2 ist, bevorzugt 1:0,5 bis 1:1,9. Bevorzugt wird ein Mol-Verhältnis von 1 : 0,9 bis 1 : 1,5 eingesetzt, insbesondere von 1:1,1, und insbesondere von etwa 1:1.

Es können die stabilen Amin-Salze der ACE-Hemmer-Dicarbonsäuren in der Matrix-Lösung bzw. Suspension in-situ gebildet werden, indem die entsprechenden organischen Amine und die ACE-Hemmer-Dicarbonsäuren zusammen in die Matrix eingearbeitet werden.

Die stabilen Amin-Salze der ACE-Hemmer-Dicarbonsäuren können aber auch direkt in die Matrix eingebracht werden.

Die erfindungsgemäßen Amin-Salze der ACE-Hemmer-Dicarbonsäuren können in der Polymer Matrix gelöst und/oder in Form von Emulsionströpfchen vorliegen.

In dem erfindungsgemäßen transdermalen therapeutischen System kann mindestens ein stabiles Alkaliverbindungs-Salz einer ACE-Hemmer-Dicarbonsäure, das auf einer Reaktion einer Alkaliverbindung mit einer ACE-Hemmer-Dicarbonsäure basiert, verwendet werden. Als Alkaliverbindung werden bevorzugt Verbindungen verwendet, die ein Alkalimetallkation umfassen. Beispiele für geeignete Alkalimetallkationen sind Lithium-, Natrium-, Kalium-, Caesium- oder Rubidiumkationen, von denen Lithium-, Natrium- und Kaliumkationen besonders bevorzugt sind. Auch Ammonium (NH₄⁺) ist als ein Kation bevorzugt (Pseudo-Alkalimetallkation). Bevorzugt hat das Gegenanion der Alkaliverbindung, insbesondere des Alkalimetallkations, Protonenakzeptoreigenschaften, und die Alkaliverbindung ist besonders bevorzugt ein Alkalimetallkationen umfassendes Salz einer starken oder schwachen, anorganischen oder organischen Säure. Bevorzugte Beispiele sind Alkaliverbindungen wie Alkalimetallhydroxide, wie zum Beispiel Lithiumhydroxid, Natriumhydroxid oder Kaliumhydroxid, Alkalimetallalkoholate, wie zum Beispiel Lithiummethanolat, Lithiumethanolat, Natriummethanolat, Natriumethanolat, Kaliummethanolat oder Kaliumethanolat, oder Alkalimetallcarbonate, wie zum Beispiel Natriumcarbonat oder Kaliumcarbonat, oder Alkalimetallhydrogencarbonate, wie zum Beispiel Natriumhydrogencarbonat oder Kaliumhydrogencarbonat, oder Alkalimetalltartrate, wie zum Beispiel Natriumtartrat oder Kaliumtartrat, oder Alkalimetallmaleate, wie zum Beispiel Natriummaleat oder Kaliummaleat. Als Ammonium-Verbindung bevorzugt ist Ammoniumhydroxid. Die Alkaliverbindung kann rein oder in Mischung mit einer geeigneten anderen Komponente, oder einem Lösungsmittel eingesetzt werden.

Das erfindungsgemäße transdermale therapeutische System kann als Wirkstoff einen oder mehrere der ACE-Hemmer enthalten, deren aktive Metaboliten eine Dicarbonsäure darstellen, wie z.B. Imidapril, Fosinopril, Moexipril, Perindopril, Spirapril, Benazepril, Cilazapril, Lisinopril, Quinapril, Enalapril, Delapril, Ramipril und/ oder Trandolapril. Unter einer Dicarbonsäure eines ACE-Hemmers versteht man den aktiven Metaboliten des ACE-Hemmers, bei welchem die im Molekül vorhandene Carbonsäureester-Gruppierung verseift wurde. Demnach zählen zu den ACE-Hemmer-Dicarbonsäure z.B. Imidaprilat, Fosinoprilat, Moexiprilat, Perindoprilat, Spiraprilat, Benazeprilat, Cilazaprilat, Lisinoprilat, Quinaprilat, Enalaprilat, Delaprilat, Ramiprilat und/ oder Trandolaprilat. Bevorzugt werden die Alkaliverbindungs-Salze und/oder die Amine von Trandolaprilat und/ oder Ramiprilat als Wirkstoffkomponenten verwendet.

Bei dem erfindungsgemäßen Alkaliverbindungs-Salz handelt es sich um ein Salz, bevorzugt ein Mono-Salz, in welchem das Mol-Verhältnis von ACE-Hemmer-Dicarbonsäure zu Alkaliverbindung kleiner als 1:2, insbesondere 1:0,5 bis 1:<2 ist, weiter bevorzugt 1:0,5 bis 1:1,9. Bevorzugt wird ein Mol-Verhältnis von 1 : 0,9 bis 1 : 1,5 eingesetzt, weiter bevorzugt von 1:1,1, und insbesondere von etwa 1 : 1.

Es können die stabilen Alkaliverbindungs-Salze der ACE-Hemmer-Dicarbonsäuren in der Matrix-Lösung bzw. Suspension in-situ gebildet werden, indem die entsprechenden Alkaliverbindungen und die ACE-Hemmer-Dicarbonsäuren zusammen in die Matrix-Lösung bzw. Suspension eingearbeitet werden.

Die stabilen Alkaliverbindungs-Salze der ACE-Hemmer-Dicarbonsäuren können aber auch direkt in die Matrix eingebracht werden.

Die erfindungsgemäßen Alkaliverbindungs-Salze der ACE-Hemmer-Dicarbonsäuren können in der Polymer Matrix gelöst und/oder in Form von Emulsionströpfchen vorliegen.

Besonders bevorzugt ist die Matrix des erfindungsgemäßen transdermalen therapeutischen Systems eine nicht-wässerige Matrix, das heißt eine Matrix, deren Gehalt bzw. Restgehalt an Wasser weniger als 2 Gew.%, bevorzugt weniger als 1 Gew.%, weiter bevorzugt weniger als 0,9 und vorzugsweise etwa 0,7 Gew.% und weniger, bezogen auf das Matrixgewicht, beträgt.

Der Gehalt an ACE-Hemmer-Dicarbonsäuren kann 2-35 Gew.%, insbesondere 10 -25 Gew.%, bezogen auf das Matrixgewicht, betragen.

Für die Haftklebeschicht kann man ein druckempfindliches Klebemittel beispielsweise auf Polyurethanbasis, Polyisobutylenbasis, Polyvinyletherbasis, Polyacrylatbasis, Silikonbasis, Styrol-Block-Copolymerbasis (z.B. Styrol-Isopren-Styrol-Block-Copolymer (SIS) oder Styrol-Butadien-Styrol-Block-Copolymer) oder ein Gemisch aus diesen wählen. Bevorzugt werden Kleber auf Acrylat- oder Polyisobutylenbasis verwendet.

Für die Matrix werden die medizinisch üblichen Matrixbildner wie Polyacrylat, Polyisobutylen, Silicone, Styrol-BlockCopolymere (z.B. Styrol-Isopren-Styrol-Block-Coploymer (SIS)) oder ein Gemisch aus diesen, verwendet. Bevorzugt wird eine selbstklebende Matrix aus Polyacrylat verwendet, wobei Matrixbildner und Klebemittel eins sind.

Die Amin-Salze der ACE-Hemmer-Dicarbonsäuren werden aus dem Matrixbildner, beispielsweise einem Polyacrylat, mit einem hohen Flux abgegeben. Unter einem hohen Flux versteht man für Trandolaprilat 0.25 bis 25 µg/cm²/h, bevorzugt 0.5 bis 5 µg/cm²/h. Für Ramiprilat versteht man unter einem hohen Flux 0.6 bis 63 µg/cm²/h, bevorzugt 1.2 bis 12 µg/cm²/h.

Bei den Klebemitteln auf Polyacrylatbasis kann es sich um ein beliebiges Homopolymer, Copolymer oder Terpolymer handeln, enthaltend oder bestehend aus verschiedenen Acrylsäurederivaten.

So können die Polyacrylate Polymere eines oder mehrerer Monomere von Acrylsäuren und anderen copolymerisierbaren Monomeren sein. Außerdem können die Polyacrylate Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat-, Alkylmethacrylat- oder Acrylamid-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat aufgeführt, die alleine oder in Mischungen polymerisiert sein können.

Zusätzlich können funktionelle Monomere, die mit den oben genannten Acrylaten, copolymerisierbar sind, wie beispielsweise Acrylsäure, Methacrylsäure, Maleinsäure, Maleinanhydrid, Hydroxyethylacrylat, Vinylacetat, Hydroxypropylacrylat, Acrylamid, Dimethylacrylamid, tert.-Octylacrylamid, Acrylnitril, Dimethylaminoethylacrylat, Dimethylaminoethylmethacrylat, tert.-Butylaminoethylacrylat, tert.-Butylaminoethylmethacrylat, Methoxyethylacrylat und Methoxyethylmethacrylat, zur Copolymerisierung eingesetzt werden.

Weitere Einzelheiten und Beispiele für druckempfindliche Acrylate, welche für die Erfindung geeignet sind, sind in Satas Handbook of Pressure Sensitive Adhesive Technology "Acrylic Adhesives", 3rd ed., (D. Satas, ed.), Van Nostrand Reinhold, New York (1999) beschrieben.

Der Gehalt an Klebemitteln der selbstklebenden Matrix kann 20 - 90 Gew.%, 30 - 80 Gew.%, insbesondere 40 - 60 Gew. %, bezogen auf das Matrixgewicht, betragen.

Für die abziehbare Schutzschicht kommen Polyester, Polyethylen, Polypropylen, Polysiloxan (beispielsweise mit einer fluorosilikonisierten Beschichtung), Polyacrylat, Ethylenvinylacetat, Polyurethan, thermoplastisches Polyurethan, Polyisobuten oder Papier, meistens mit Silikon- und /oder Polyethylen beschichtet, oder ein Gemisch aus diesen in Betracht.

Als undurchlässige Deckschicht kommen Folien aus Acrylat, Acrylonitril-Butadien-Styrol, Acrylonitril (Methyl Methacrylat) Copolymer, Acrylonitril Copolymer, Ethylen Ethyl Acrylat, Ethylen Methyl Acrylat, Ethylen Vinyl Acetat, Ethylen Vinyl Acetat Copolymer, Ethylen Vinylalkohol Polymer-, Ionomere, Nylon (Polyamid), Nylon (Polyamid) Copolymer, Polybutylen, Polycarbonat , Polyester, Polyethylenterephthalat, thermoplastisches Polyester Copolymer, Polyethylen Copolymer (high density), Polyethylen (high-molecular-weight, highdensity), Polyethylen (intermediate-molecular-weight, highdensity), Polyethylen (linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/ oder Styrol-Acrylonitril in Frage, die bei Bedarf metallisiert oder pigmentiert werden können. Als für den Wirkstoff undurchlässige Deckschicht werden Polyurethan, Ethylenvinylalkohol-Polymer und Polyester bevorzugt.

Als Permeätionsförderer lassen sich gegebenenfalls gesättigte und/ oder ungesättigte Fettalkohole mit jeweils 8 bis 18 C-Atomen; Teebaumöl; gesättigte und/ oder ungesättigte cyclische Ketone; Alkyl- Methylsulfoxide; gesättigte und/ oder ungesättigte Fettsäuren mit jeweils 8 bis 18 C-Atomen; deren Ester und Salze; natürliches Vitamin E; synthetisches Vitamin E und/ oder Vitamin E- Derivate; Sorbitanfettsäureester und ethoxylierte Sorbitanfettsäureester; Azone (Laurocapram); 1-Alkylpyrrolidon; Blockcopolymere von Polyethylenglykol und Dimethylsiloxan mit kationischer Gruppe an einem Ende; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit > 8 C-Atomen; nichtionische Tenside, z.B. Laurylether, Ester von Polyoxyethylen; Dimethyl(arylimino)sulfuran; Gemisch aus Ölsäureanaloga und Propylenglykol; Gemisch aus Padimat O, Oktylsalicylat, Isopropylmyristat, Isopropylpalmitat, Oktylmethoxycinnimat, Laurocapram; hochdisperses Siliziumdioxid (Aerosil^{®}); Polyoxyethylen-7-glycerol-monococoat (Cetiol^{®} HE); 2-Octyldodecanol (Eutanol^{®} G) oder ein Gemisch aus verschiedenen Einzelkomponenten verwenden. Es wird in dem erfindungsgemäßen transdermalen therapeutischen System Polyoxyethylen-7-glycerol-monococoat (Cetiol^{®} HE) oder 2-Octyldodecanol (Eutanol^{®} G) als Permeationsförderer bevorzugt.

Als Füllstoffe können folgende Substanzen oder deren Mischungen verwendet werden: Metalloxide wie Zink-, Magnesium-, Calcium- oder Titanoxid, anorganische Salze wie Calciumcarbonat, Magnesiumcarbonat, Natriumcarbonat, Calciumsulfat, Magnesiumsulfat oder Calciumphospat, Tonkomponenten wie Talk, Kaolin, Bentonit oder Polymerfüllstoffe.

Als Füllstoffe können insbesondere Zinkoxid und/oder Aerosil, eingesetzt werden.

Das erfindungsgemäße transdermale therapeutische System mit einer selbstklebenden Matrix kann folgendermaßen aufgebaut sein. Die oberste Schicht stellt die für den Wirkstoff undurchlässige Deckschicht dar. Dann folgt die selbstklebende Matrixschicht, die den Wirkstoff und fakultative Permeationshemmer und/oder Füllstoffe enthält. Der Matrixbildner ist in diesem Fall der Haftkleber. Den Abschluß bildet eine abziehbare Schutzschicht.

Alternativ kann das erfindungsgemäße transdermale therapeutische System eine nicht selbstklebende Matrixschicht enthalten, die mit einer separaten Haftkleberschicht versehen ist.

Besonders bevorzugt umfasst das erfindungsgemäße transdermale therapeutische System eine wirkstoffhaltige Matrixschicht, die eine nicht-wässerige Matrixschicht ist, das heißt eine Matrixschicht, deren Gehalt bzw. Restgehalt an Wasser weniger als 2 Gew.%, bevorzugt weniger als 1 Gew.%, weiter bevorzugt weniger als 0,9 Gew.-% und vorzugsweise etwa 0,7 Gew.% oder weniger, bezogen auf das Matrixgewicht, beträgt.

Die Erfindung wird durch nachstehende Beispiele näher erläutert.

Dabei werden insbesondere die folgenden Komponenten verwendet: Als ein Kleber auf Polyacrylatbasis: Durotak^{®} 78-2353; als ein Kleber auf Basis eines Copolymers aus Acrylaten und Vinylacetat: Durotak^{®} 87-4098; als ein Kleber auf Basis von Styrol-Butadien-Styrol-Block-Copolymer: Durotak^{®} 87-6173; als ein Kleber auf Basis von Polyisobutylen: Durotak^{®} 87-6430; als ein Siliconkleber: BioPSA Hex 7-4302; Als Permeationsförderer: Cetiol^{®} HE: Polyoxyethylen-7-glycerol-monococoat.

### Beispiel 1:

Zusammensetzung einer erfindungsgemäßen selbstklebenden Matrix für ein TTS mit einem Mono-salz aus Trandolaprilat und Trometamol

| Inhaltsstoffe | Gehalt in Gew.% |
|---|---|
| Trandolaprilat | 10 |
| Trometamol | 4,5 |
| Cetiol^{®} HE | 5 |
| Durotak^{®} 87-2353 | 80,5 |

Die Gewichtsprozente beziehen sich auf das Matrixgewicht. Das stöchiometrische Verhältnis von Trandolaprilat zu Trometamol beträgt 1: 1,5.

### Herstellungsprozeß:

In einem Rührgefäß werden Trandolaprilat und Trometamol in einem geeigneten Lösemittel gelöst. Anschließend wird zuerst Cetiol^{®}HE und dann der Kleber (Durotak^{®}) zugegeben und homogenisiert. Diese Mischung wird auf eine abziehbare Folie aufgetragen und getrocknet. Auf diese Matrix wird dann eine PET-Folie als wirkstoffundurchlässige Deckschicht aufgebracht. Danach werden die TTS ausgestanzt und in Sachets verpackt.

### Beispiel 2:

Zusammensetzung einer erfindungsgemäßen selbstklebenden Matrix für ein TTS mit einem Mono-salz aus Trandolaprilat und Trometamol

| Inhaltsstoffe | Gehalt in Gew.% |
|---|---|
| Trandolaprilat | 10 |
| Trometamol | 3 |
| Cetiol^{®} HE | 5 |
| Durotak^{®} 87-2353 | 82 |

Die Gewichtsprozente beziehen sich auf das Matrixgewicht. Das stöchiometrische Verhältnis von Trandolaprilat zu Trometamol beträgt 1: 1.

Die Herstellung erfolgt analog Beispiel 1.

### Stabilität:

Stabilität eines transdermalen therapeutischen System mit einem Mono-salz von Trandolaprilat mit Trometamol (Verhältnis von Trandolaprilat zu Trometamol ist 1: 1)

| Lagerdauer | Lagerbedingungen | Summe der Abbauprodukte [%] bezogen auf den Wirkstoff |
|---|---|---|
| 0 Monate | nicht kontrolliert | 1.0 |
| 3 Monate | 25°C/60% rF | 0.9 |
| 3 Monate | 40°C/75% rF | 1.1 |

Wie man der Tabelle entnehmen kann, zeigt ein TTS mit einem Mono-Salz von Trandolaprilat eine hohe Stabilität.

### Hautirritationen:

Ein transdermales therapeutischen System mit einem Mono-salz von Trandolaprilat mit Trometamol (Verhältnis von Trandolaprilat zu Trometamol ist 1: 1,1) zeigt einen durchschnittlichen Wert für Erytheme - eine entzündliche Rötung der Haut bedingt durch Hyperämie - von 1.

Placebo zeigt einen durchschnittlichen Wert für Erytheme von 1.

Gemäß DRAIZE (Appraisal of the Safety of Chemicals in Food, Drugs and Cosmetics, Association of Food and Drug Officals of the United States, Austin, Texas, 1959) können Erytheme wie folgt klassifiziert werden.

| **Erythem** | **Wert** |
|---|---|
| Keine Erythembildung | 0 |
| Sehr geringe Erythembildung (kaum bemerkbar) | 1 |
| Deutliche Rötung | 2 |
| Moderate bis schwere Erythembildung | 3 |
| Schwere Erythembildung (starke Rötung) oder Ätzschorfbildung (tiefe Verletzung) | 4 |

Demnach zeigt ein transdermales therapeutisches System mit einem Mono-Salz von Trandolaprilat einen kleinen Erythem-Wert, d.h. keine Hautirritation. Es zeigt gegenüber Placebo keinen Unterschied.

### pH-Wert:

Zur Bestimmung des pH-Wertes der transdermalen therapeutischen Systeme wurden 10 cm² grosse Laminatausschnitte in 25 ml Wasser hinreichend lange geschüttelt, d.h. bis zum Einstellen eines konstanten pH-Wertes. Der pH-Wert wurde mit einem üblichen pH-Meter bestimmt.

| ACE-Hemmer | organisches Amin | Verhältnis ACE-Hemmer:Amin | pH-Wert |
|---|---|---|---|
| Trandolaprilat | Trometamol | 1 : 2 | 7,8 |
| Trandolaprilat | Trometamol | 1 : 1,1 | 6,7 |

### Beispiel 3:

Zusammensetzung einer erfindungsgemäßen selbstklebenden Matrix für ein TTS mit einem Salz aus Ramiprilat und Natriumhydroxid (NaOH) in Polyacrylatmatrix:

| Inhaltsstoff | Gehalt in Gew.% |
|---|---|
| Ramiprilat | 10 |
| NaOH | 1,3 |
| Cetiol^{®} HE | 5 |
| Durotak^{®} 87-4098 | 83,7 |

Die Gewichtsprozente beziehen sich auf das Matrixgewicht. Das stöchiometrische Verhältnis von Ramiprilat zu Natriumhydroxid (NaOH) beträgt 1: 1,3.

Die Herstellung erfolgt analog Beispiel 1.

### Beispiel 4:

Zusammensetzung einer erfindungsgemäßen selbstklebenden Matrix für ein TTS mit einem Salz aus Trandolaprilat und Octylamin in Siliconmatrix:

| Inhaltsstoff | Gehalt in Gew.% |
|---|---|
| Trandolaprilat | 10 |
| Octylamin | 4,8 |
| BioPSA Hex 7-4302 | 85,2 |

Die Gewichtsprozente beziehen sich auf das Matrixgewicht. Das stöchiometrische Verhältnis von Trandolaprilat zu Octylamin beträgt 1: 1,5:

Die Herstellung erfolgt analog Beispiel 1 unter Verwendung einer fluorosiliconisierten Abziehfölie.

Der pH-Wert des transdermalen therapeutischen Systems (Bestimmung s. Beispiel 2) beträgt 5,0.

### Beispiel 5:

Zusammensetzung einer erfindungsgemäßen selbstklebenden Matrix für ein TTS mit einem Salz aus Trandolaprilat und Dodecylamin in einer Styrol-Block-Copolymermatrix oder PIB-Matrix:

| Inhaltsstoff | Gehalt in Gew.% |
|---|---|
| Trandolaprilat | 10 |
| Dodecylamin | 5,5 |
| Isopropylpalmitat | 5 |
| Durotak^{®} 87-6173 oder Durotak^{®} 87-6430 | 79,5 |

Die Gewichtsprozente beziehen sich auf das Matrixgewicht. Das stöchiometrische Verhältnis von Trandolaprilat zu Dodecylamin beträgt 1: 1,2.

Die Herstellung erfolgt analog Beispiel 1.

### Beispiel 6:

Zusammensetzung einer erfindungsgemäßen selbstklebenden Matrix für ein TTS mit einem Salz aus Trandolaprilat und Butylamin in Siliconmatrix:

| Inhaltsstoff | Gehalt in Gew.% |
|---|---|
| Trandolaprilat | 10 |
| Butylamin | 2,7 |
| BioPSA Hex 7-4302 | 87,3 |

Die Gewichtsprozente beziehen sich auf das Matrixgew-icht. Das stöchiometrische Verhältnis von Trandolaprilat zu Butylamin beträgt 1: 1,5.

Die Herstellung erfolgt analog Beispiel 1 unter Verwendung einer fluorosiliconisierten Abziehfolie.

## Patentansprüche

1. Transdermales therapeutisches System mit mindestens einem Salz einer ACE-Hemmer-Dicarbonsäure mit mindestens einem organischen Amin, wobei das Salz ein Molverhältnis von ACE-Hemmer-Dicarbonsäure : Amin von 1 : kleiner 2 aufweist.

2. Transdermales therapeutisches System nach Anspruch 1 mit einem Monoamin als organischem Amin.

3. Transdermales therapeutisches System nach Anspruch 1 und/oder 2 mit einem primären Amin, einem sekundären Amin oder einen tertiären Amin als organischem Amin.

4. Transdermales therapeutisches System nach mindestens einem der vorhergehenden Ansprüche mit mindestens einem Salz einer ACE-Hemmer-Dicarbonsäure aus der Gruppe der Dicarbonsäuren von Imidapril, Fosinopril, Moexipril, Perindopril, Spirapril, Benazepril, Cilazapril, Lisinopril, Quinapril, Enalapril, Delapril, Ramipril und Trandolapril.

5. Transdermales therapeutisches System nach mindestens einem der vorhergehenden Ansprüche 1 bis 4 mit
- einer wirkstoffundurchlässigen Deckschicht,
- einer oder mehreren wirkstoffhaltigen selbstklebenden Matrixschichten und
- einer abziehbaren Schutzschicht.

6. Transdermales therapeutisches System nach mindestens einem der vorhergehenden Ansprüche 1 bis 4 mit
- einer wirkstoffundurchlässigen Deckschicht,
- einer oder mehreren wirkstoffhaltigen Matrixschichten,
- die applikationsseitig mit einer Haftkleberschicht versehen ist (sind), und
- einer abziehbaren Schutzschicht.

7. Transdermales therapeutisches System nach mindestens einem der vorhergehenden Ansprüche 1 bis 6, bei dem der oder die Wirkstoffe in der Matrix gelöst und/oder in Form von Emulsionströpfchen vorliegen.

8. Transdermales therapeutisches System nach mindestens einem der vorhergehenden Ansprüche 1 bis 7, wobei der Gehalt an ACE-Hemmer-Dicarbonsäure 2 bis 35 Gew.-% beträgt, bezogen auf das Matrixgewicht.

9. Transdermales therapeutisches System nach einem der vorhergehenden Ansprüche 1 bis 8 mit einem druckempfindlichen Klebemittel auf Basis Polyurethan, Polyisobutylen, Polyvinylether, Polyacrylat, Silikon, Styrol-Block-Copolymer oder eines Gemischs derselben.

10. Transdermales therapeutisches System nach einem der vorhergehenden Ansprüche 1 bis 9 mit einem Matrixbildner aus der Gruppe von Polyacrylat, Polyisobutylen, Silicon, Styrol-Block-Copolymer oder einem ihrer Gemische.

11. Transdermales therapeutisches System nach einem der vorhergehenden Ansprüche 1 bis 10 mit einem Haftkleber und/oder einer Matrix auf Polyacrylatbasis, bei der es sich um ein Homopolymer, Copolymer oder Terpolymer handelt.

12. Transdermales therapeutisches System nach einem der vorhergehenden Ansprüche 1 bis 11 mit einem Permeationsförderer, ausgewählt aus der Gruppe:
gesättigte und/oder ungesättigte Fettalkohole mit jeweils 8 bis 18 C-Atomen;
Teebaumöl;
gesättigte und/oder ungesättigte cyclische Ketone;
Alkyl-Methylsulfoxide;
gesättigte und/oder ungesättigte Fettsäuren mit jeweils 8 bis 18 C-Atomen;
Ester gesättigter und/oder ungesättigter Fettsäuren mit jeweils 8 bis 18 C-Atomen;
Salze gesättigter und/oder ungesättigter Fettsäuren mit jeweils 8 bis 18 C-Atomen;
natürliches Vitamin E;
synthetisches Vitamin E und/oder Vitamin E- Derivate;
Sorbitanfettsäureester;
ethoxylierte Sorbitanfettsäureester;
Azone, insbesondere Laurocapram;
1-Alkylpyrrolidon;
Blockcopolymere von Polyethylenglykol und Dimethylsiloxan mit kationischer Gruppe an einem Ende;
Polyoxyethylen-10-stearylether;
Gemisch aus Polyoxyethylen-10-stearylether und
Glyceryldilaurat;
Dodecyl-2-(N,N-dimethylamino)-propanoltetradecanoat und/oder Dodecyl-2-(N,N-dimethylamino)-propionat;
N-Acetylprolinatester (N-Acetyl-pyrrolidon-2-carbonsäureester) mit > 8 C-Atomen;
nichtionische Tenside, insbesondere Laurylether;
Ester von Polyoxyethylen;
Dimethyl(arylimino)aulfuran;
Gemisch aus Ölsäureanalogon(a) und Propylenglykol;
Gemisch aus Padimat O Octylsalicylat, Isopropylmyristat, Isopropylpalmitat, Octylmethoxycinnamat, Laurocapram;
hochdisperses Siliziumdioxid (Aerosil^{®});
Polyoxyethylen-7-glycerol-monococoat (Cetiol^{®} HE);
2-Octyldodecanol (Eutanol^{®} G);
und deren Gemischen.

13. Transdermales therapeutisches System nach einem der vorhergehenden Ansprüche 1 bis 12, wobei der Gehalt an Klebemittel der selbstklebenden Matrix 20 bis 90 Gew.-%, bevorzugt 30 bis 80 Gew.-% und insbesondere 40 bis 60 Gew.-% beträgt, Rest Wirkstoff(e), fakultativer Permeatonsförderer und fakultativer Füllstoff, jeweils bezogen auf das Matrixgewicht.

14. Verfahren zur Herstellung eines transdermalen therapeutischen Systems gemäß einem der vorhergehenden Ansprüche 1 bis 13, bei dem
a) das (die) organische(n) Amin(e) und die ACE-Hemmer-Dicarbonsäure(n) zusammen in die Matrix-Lösung bzw. Suspension eingearbeitet werden; und
b) das (die) Amin-Salz(e) in der Matrix-Lösung bzw. Suspension in-situ gebildet wird (werden).

15. Verfahren zur Herstellung eines transdermalen therapeutischen Systems gemäß einem der Ansprüche 1 bis 13, bei dem das (die) Amin-Salz(e) der ACE-Hemmer-Dicarbonsäure(n) direkt in die Matrix eingebracht wird (werden).

## Claims

1. Transdermal therapeutic system with at least one salt of an ACE inhibitor dicarboxylic acid with at least one organic amine, wherein the salt has a molar ratio of ACE inhibitor dicarboxylic acid: amine of 1 : less than 2.

2. Transdermal therapeutic system according to claim 1 with a monoamine as organic amine.

3. Transdermal therapeutic system according to claim 1 and/or 2 with a primary amine, a secondary amine or a tertiary amine as organic amine.

4. Transdermal therapeutic system according to at least one of the preceding claims with at least one salt of an ACE inhibitor dicarboxylic acid from the group of the dicarboxylic acids of imidapril, fosinopril, moexipril, perindopril, spirapril, benazepril, cilazapril, lisinopril, quinapril, enalapril, delapril, ramipril and trandolapril.

5. Transdermal therapeutic system according to at least one of the preceding claims 1 to 4 with
- a top layer impermeable to active ingredient,
- one or more active ingredient-containing self-adhesive matrix layers and
- a peel-off protective layer.

6. Transdermal therapeutic system according to at least one of the preceding claims 1 to 4 with
- a top layer impermeable to active ingredient,
- one or more active ingredient-containing matrix layers,
- which is(are) provided on the application side with a layer of contact adhesive, and
- a peel-off protective layer.

7. Transdermal therapeutic system according to at least one of the preceding claims 1 to 6, in which the active ingredient or the active ingredients is/are dissolved and/or is/are present in the form of droplets of emulsion in the matrix.

8. Transdermal therapeutic system according to at least one of the preceding claims 1 to 7, wherein the content of ACE inhibitor dicarboxylic acid is from 2 to 35% by weight, based on the weight of the matrix.

9. Transdermal therapeutic system according to any one of the preceding claims 1 to 8 with a pressure-sensitive adhesive based on polyurethane, polyisobutylene, polyvinyl ether, polyacrylate, silicone, styrene block copolymer or a mixture thereof.

10. Transdermal therapeutic system according to any one of the preceding claims 1 to 9 with a matrix former from the group of polyacrylate, polyisobutylene, silicone, styrene block copolymer or a mixture thereof.

11. Transdermal therapeutic system according to any one of the preceding claims 1 to 10 with a contact adhesive and/or a matrix based on polyacrylate, which may be a homopolymer, copolymer or terpolymer.

12. Transdermal therapeutic system according to any one of the preceding claims 1 to 11 with a permeation enhancer selected from the group formed by
saturated and/or unsaturated fatty alcohols each having from 8 to 18 C atoms;
tea tree oil;
saturated and/or unsaturated cyclic ketones;
alkyl methyl sulphoxides;
saturated and/or unsaturated fatty acids each having from 8 to 18 C atoms;
esters of saturated and/or unsaturated fatty acids each having from 8 to 18 C atoms;
salts of saturated and/or unsaturated fatty acids each having from 8 to 18 C atoms;
natural vitamin E;
synthetic vitamin E and/or vitamin E derivatives;
sorbitan fatty acid esters;
ethoxylated sorbitan fatty acid esters;
azones, especially laurocapram;
1-alkylpyrrolidone;
block copolymers of polyethylene glycol and dimethylsiloxane having a cationic group at one end;
polyoxyethylene-10 stearyl ether;
a mixture of polyoxyethylene-10 stearyl ether and glyceryl dilaurate;
dodecyl-2-(N,N-dimethylamino)-propanoltetradecanoate and/or dodecyl 2-(N,N-dimethylamino)-propionate;
N-acetylprolinate esters (N-acetyl-pyrrolidone-2-carboxylic acid esters) having > 8 C atoms;
non-ionic surfactants, especially lauryl ether;
esters of polyoxyethylene;
dimethyl(arylimino)sulphuran;
a mixture of oleic acid analogue(s) and propylene glycol;
a mixture from padimate O, octyl salicylate, isopropyl myristate, isopropyl palmitate, octyl methoxycinnamate, laurocapram;
highly disperse silicon dioxide (Aerosil^{®}); polyoxyethylene-7-glycerol monococoate (Cetiol^{®} HE); 2-octyldodecanol (Eutanol^{®} G);
and mixtures thereof.

13. Transdermal therapeutic system according to any one of the preceding claims 1 to 12, wherein the content of adhesive in the self-adhesive matrix is from 20 to 90% by weight, preferably from 30 to 80% by weight and especially from 40 to 60% by weight, with the remainder being active ingredient(s), optional permeation enhancer and optional filler, in each case based on the weight of the matrix.

14. Method of producing a transdermal therapeutic system according to any one of the preceding claims 1 to 13, in which
(a) the organic amine(s) and the ACE inhibitor dicarboxylic acid(s) are together incorporated into the matrix solution or suspension; and
(b) the amine salt(s) is (are) formed in situ in the matrix solution or suspension.

15. Method of producing a transdermal therapeutic system according to any one of claims 1 to 13, in which the amine salt(s) of the ACE inhibitor dicarboxylic acid(s) is (are) introduced into the matrix directly.

## Revendications

1. Système transdermique thérapeutique avec au moins un sel d'un acide dicarboxylique inhibiteur d'ACE avec au moins une amine organique, où le sel présente un rapport molaire acide dicarboxylique inhibiteur d'ACE:amine de 1 : moins de 2.

2. Système transdermique thérapeutique selon la revendication 1, avec une monoamine comme amine organique.

3. Système transdermique thérapeutique selon la revendication 1 et/ou 2, avec une amine primaire, une amine secondaire ou une amine tertiaire comme amine organique.

4. Système transdermique thérapeutique selon au moins l'une des revendications précédentes, avec au moins un sel d'un acide dicarboxylique d'inhibiteur d'ACE du groupe des acides carboxyliques imidapril, fosinorpil, moexipril, périndopril, spirapril, bénazépril, cilazapril, lisinopril, quinapril, énalapril, délapril, ramipril et trandolapril.

5. Système transdermique thérapeutique selon au moins l'une des revendications précédentes 1 à 4, avec
- une couche de couverture imperméable pour l'agent actif,
- une ou plusieurs couches matricielles autocollantes, contenant l'agent actif, et
- une couche protectrice détachable.

6. Système transdermique thérapeutique selon au moins l'une des revendications précédentes 1 à 4, avec
- une couche de couverture imperméable pour l'agent actif,
- une ou plusieurs couches matricielles autocollantes, contenant l'agent actif,
- qui sont munies, côté application, d'une couche autoadhésive, et
- une couche protectrice détachable.

7. Système transdermique thérapeutique selon au moins l'une des revendications précédentes 1 à 6, dans lequel le ou les agents actifs sont présents sous forme dissoute dans la matrice et/ou sous forme de gouttelettes d'émulsion.

8. Système transdermique thérapeutique selon au moins l'une des revendications précédentes 1 à 7, où la teneur en acide dicarboxylique inhibiteur d'ACE se situe dans l'intervalle allant de 2 à 35 % en poids, sur base du poids de la matrice.

9. Système transdermique thérapeutique selon l'une des revendications précédentes 1 à 8, avec une colle autocollante à base d'un polyuréthanne, un polyisobutylène, un polyvinyléther, un polyacrylate, une silicone, un copolymère séquencé de styrène ou un mélange de ceux-ci.

10. Système transdermique thérapeutique selon l'une des revendications précédentes 1 à 9, avec un agent formant matrice choisi parmi le groupe des polyacrylate, polyisobutylène, silicone, copolymère séquencé de styrène ou un mélange de ceux-ci.

11. Système transdermique thérapeutique selon l'une des revendications précédentes 1 à 10, avec un autocollant et/ou une matrice à base d'un polyacrylate, qui consiste en un homopolymère, un copolymère ou un terpolymère.

12. Système transdermique thérapeutique selon l'une des revendications précédentes 1 à 11, avec un accélérateur de perméation, choisi parmi le groupe consistant en :
des alcools gras saturés et/ou insaturés avec chaque fois 8 à 18 atomes C ;
l'huile de théier ;
des cétones cycliques saturées et/ou insaturées ;
un alkylméthylsulfoxyde ;
des acides gras saturés et/ou insaturés avec chaque fois 8 à 18 atomes C ;
des esters d'acides gras saturés et/ou insaturés avec chaque fois 8 à 18 atomes C ;
des sels d'acides gras saturés et/ou insaturés avec chaque fois 8 à 18 atomes C ;
la vitamine E naturelle ;
la vitamine E synthétique et/ou des dérivés de vitamine E ;
des esters d'acide gras du sorbitan ;
des esters d'acide gras du sorbitan éthoxylés ;
des azones, en particulier le laurocaprame ;
une 1-alkylpyrrolidone ;
des copolymères séquencés de polyéthylèneglycol et de diméthylsiloxane avec des groupes cationiques à une extrémité ;
un polyoxyéthylène-10-stéaryléther ;
un mélange de polyoxyéthylène-10-stéaryléther et de dilaurate de glycéryle ;
le tétradécanoate de dodécyl-2-(N,N-diméthyl-amino)propanol et/ou le 2-(N,N-diméthylamino)propionate de dodécyle ;
un ester N-acétylprolinate (ester d'acide N-acétylpyrrolidone-2-carboxylique) avec > 8 atomes C ;
des tensioactifs non ioniques, en particulier un lauryléther ;
des esters de polyoxyéthylène ;
le diméthyl(arylimino)sulfurane ;
un mélange d'analogue(s) d'acide oléique et de propylèneglycol ;
un mélange de padimate O, de salicylate d'octyle, de myristate d'isopropyle, de palmitate d'isopropyle, de méthoxycinnamate d'octyle, de laurocaprame ;
le dioxyde de silicium fortement dispersé (Aerosil®) ;
le polyoxyéthylène-7-glycérol-monococoate (Cetiol® HE) ;
le 2-octyldodécanol (Eutanol® G) ;
et leurs mélanges.

13. Système transdermique thérapeutique selon l'une des revendications précédentes 1 à 12, où la teneur en agent collant de la matrice autocollante se situe dans l'intervalle allant de 20 à 90 % en poids, de préférence de 30 à 80 % en poids et en particulier de 40 à 60 % en poids, le reste étant le ou les agents actifs, l'accélérateur de perméation facultatif et la charge facultative, chaque fois sur base du poids de matrice.

14. Procédé de préparation d'un système transdermique thérapeutique selon l'une des revendications précédentes 1 à 13, dans lequel
a) la ou les amines organiques et le ou les acides dicarboxyliques inhibiteur d'ACE sont incorporés à la solution ou suspension de matrice, et
b) le ou les sels d'amines sont formés *in situ,* dans la solution ou suspension de matrice.

15. Procédé de préparation d'un système transdermique thérapeutique selon l'une des revendications précédentes 1 à 13, dans lequel le ou les sels d'amine du ou des acides dicarboxyliques inhibiteur d'ACE sont incorporés directement dans la matrice.
